(19) Europäisches Patentamt
European Patent Office
Office européen des brevets

(11) **EP 3 552 616 B1**

(12) **EUROPEAN PATENT SPECIFICATION**

(45) Date of publication and mention
of the grant of the patent:
**29.12.2021 Bulletin 2021/52**

(21) Application number: **18166386.5**

(22) Date of filing: **09.04.2018**

(51) Int Cl.:
*A61K 36/61* (2006.01)          *A61K 8/00* (2006.01)
*A61P 17/02* (2006.01)          *A61K 8/9789* (2017.01)
*A61Q 19/00* (2006.01)          *A61Q 19/02* (2006.01)
*A61Q 19/08* (2006.01)

(54) **HYDROLYSATE OF WATER EXTRACT OF SYZYGIUM SAMARANGENSE, AND PREPARATION PROCESS AND USE THEREOF**

HYDROLYSAT VON WASSEREXTRAKT AUS SYZYGIUM SAMARANGENSE, HERSTELLUNGSVERFAHREN UND VERWENDUNG DAVON

HYDROLYSAT D'EXTRAIT D'EAU DE JAMALAC ET PROCÉDÉ DE PRÉPARATION ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**16.10.2019 Bulletin 2019/42**

(73) Proprietor: MHBT Co., Ltd.
**64069 Douliou City (TW)**

(72) Inventor: **CHIANG, Chun-Hui**
**64069 Douliou City (TW)**

(74) Representative: **Regimbeau**
**20, rue de Chazelles**
**75847 Paris Cedex 17 (FR)**

(56) References cited:
**WO-A1-2005/056031     CN-B- 102 228 207**

EP 3 552 616 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE DISCLOSURE**

The present disclosure relates to a hydrolysate of water extract of *Syzygium samarangense* and a preparation process thereof. The present disclosure also relates to use of the aforesaid hydrolysate for whitening skin, enhancing the moisture-retaining capacity of skin, improving wound healing, and reducing oxidative stress.

**BACKGROUND**

Skin is the largest protective barrier for the human body, and is able to prevent water loss, entering of pathogens, and damages by various harmful environments. Heavy exposure to ultraviolet (UV), ionizing radiation, drugs, or xenobiotics induces generation of reactive oxygen species (ROS) and free radicals in skin. When accumulating ROS and free radicals exceed the antioxidant capacity of cells or tissues, oxidative stress occurs. Particularly, ROS and free radicals react with cell components (including DNA, proteins, and lipids), such that skin is adversely influenced. It has been reported that ROS and free radicals not only play an important role in melanogenesis, but also lead to delay of wound healing (Kim Y.J. and Yokozawa T. (2009), Biol. Pharm. Bull., 32:1155-1159; and Kurahashi T. and Fujii J. (2009), J. Dev. Biol., 3:57-70).

Melanogenesis is a process where tyrosine in melanocytes is converted to melanin under catalysis of tyrosinase and a series of oxidation-reduction reactions. Melanogenesis is normally induced by UV radiation (particularly UV-B). When skin is exposed to UV radiation, the ROS and free radicals generated in keratinocytes induce expression of tumor protein 53 (p53), thereby giving rise to expression of the proopiomelanocortin gene (POMC gene) and releasing POMC-derived peptides such as $\alpha$-melanocyte stimulating hormone ($\alpha$-MSH) and adrenocorticotropic hormone (ACTH). Further, the tyrosine gene in melanocytes is expressed, such that a large amount of melanin accumulates on skin.

When skin damage (for example, scald, trauma, surgical injury, contusion, etc.) leads to wound, an inflammatory response is triggered at the wound site. A tremendous amount of ROS and free radicals are released to assist immune cells in fighting against pathogens. However, excess ROS and free radicals cause damage to the tissues surrounding wound. In the wound healing process, fibroblasts aggregate at the wound site to proliferate and to release growth factors, thereby resulting inangiogenesis, epithelialization, collagen remolding, and so forth. Excess ROS and free radicals not only inhibit angiogenesis, but also inactivate the enzymes involved in signal transduction pathways, such that wound healing is hindered.

In recent years, the demand for skin whitening, moisturizing, facilitation of wound healing, and antioxidation have been increasing. Particularly, use of natural ingredients, which do not cause undesired harmful side effects on skin, has become a trend for the field of aesthetic medicine. Therefore, to satisfy such great demand, researchers in the health care industry and cosmetic industry endeavor to look for safe active ingredients, which are effective in whitening, moisturizing, wound healing, and antioxidation, from traditional Chinese medicines (TCM) or plants.

*Syzygium samarangense* (common name: wax apple; Pinyin: lian wu) is a perennial evergreen tree which belongs to the family *Myrtaceae* and the genus *Syzygium,* and which has opposite simple leaves that are elliptical in shape, axillary flowers that have pale yellow corolla, and berries that are obconical in shape. *Syzygium samarangense* is distributed over Taiwan, Indonesia, Philippines, Malaysia, and so forth. The common cultivars of *Syzygium samarangense* fruits include dark red cultivars, pale red cultivars, "Black Pearl", "Black King Kong", "Black Diamond", etc. *Syzygium samarangense* fruits are frequently used. In the field of Chinese fold medicine, *Syzygium samarangense* fruits are used for lung nourishing, cough suppression, sputum reduction, blood cooling, and induction of astringency. In addition, *Syzygium samarangense* fruits are considered to be effective in relieving fever, promoting dieresis, and reducing mental strain.

The application of the *Syzygium samarangense* extract in the pharmaceutical field has been studied. For instance, as reported in the thesis written by Pei-Wen Lo from the Department of Nutrition and Food Science at Fu-Jen Catholic University ("Evaluation of the Antioxidative and Free Radical-scavenging Activity of Several Taiwan Unique Fruits"), the antioxidation efficacy regarding the *Syzygium samarangense* fruit and other types of fruits was investigated. Specifically, 14 types of fruits (fruits of *Citrus sinensis Osbeck, Citrus tankan Hayata, Citrus grandis (L) Osbeck, Citrus grandis Osbeck, Citrus micro carpa, Fragaria ananassa Duch, Hylocereus undatus, Syzygium samarangense, Diospyros kaki L., Mangifera indica L. , Carica papaya L., Zizyphusjujuba, Averrhoa carambola L.,* and *Psidium guajava L.*) were subjected to lyophilization and grinding to form powder. 10 g of powder of a respective fruit was subjected to a 6-hour extraction treatment using 100 mL of methanol, followed by a filtration treatment with a filter paper. The resulting filtrate was subjected to concentration and drying. Thus, 14 methanol extracts of fruit were obtained, and were tested for the antioxidation efficacy. The experimental results indicate that the aforesaid 14 methanol extracts of fruit exhibit different levels of activities in different tests. Particularly, via the test for the $\alpha,\alpha$-diphenyl-$\beta$-picrylhydrazyl (DPPH) radical scavenging ability, it was found that the methanol extract of a fruit material (containing a peel and a pulp) of *Syzygium*

*samarangense* has the worst DPPH radical scavenging ability. Furthermore, the result of the test for the ability to inhibit superoxide anion generation reveals that the methanol extract of the fruit material of *Syzygium samarangense* is not able to inhibit superoxide anion generation, but instead induces superoxide anion generation.

In addition, as described in the thesis written by Yi-Zhen Chen from the Institute of Cosmetic Science at Chia NanUniversity of Pharmacy & Science ("Application of Extracts from Wax Apple Flowers on Antioxidantion"), the antioxidation efficacy and tyrosinase inhibition ability regarding the *Syzygium samarangense* flower were examined. Specifically, 50 g of dry powder of a *Syzygium samarangense* flower was subjected to a one-hour extraction treatment with 500 mL of 80% methanol, and 50 g of dry powder of a *Syzygium samarangense* flower was subjected to a one-hour extraction treatment with 500 mL of water. Subsequently, a filtration treatment was conducted using a filtration paper. The resulting two filtrates were subjected to concentration and lyophilization, such that a methanol extract of *Syzygium samarangense* flower and a water extract of *Syzygium samarangense* flower were obtained. The aforesaid two extracts were tested for the antioxidation efficacy and tyrosinase inhibition activity. In most of the tests for the antioxidation efficacy (i.e. the tests for the superoxide anion scavenging ability, lipid peroxidation inhibition activity, and ferrous ion chelating ability), it was found that the antioxidation efficacy of the aforesaid water extract is better than that of the aforesaid methanol extract. However, the results show that the tyrosinase inhibition ability of the aforesaid methanol extract is significantly better than that of the water extract.

TW 1465258 discloses an ethanol extract of *Syzygium samarangense* flower (which has skin whitening and anti-aging effects) and a preparation process thereof. The aforesaid preparation process includes subjecting dry powder of *Syzygium samarangense* flower to an extraction treatment with 95% ethanol, subsequently conducting a filtration treatment, and subjecting the filtrate thus obtained to a concentration treatment. It was verfied from experiments that the aforesaid ethanol extract of *Syzygium samarangense* flower is effective in serving as an antioxidant, inhibiting tyrosinase, and facilitating collagen formation.

CN 102228207 A discloses a method for processing *Syzygium samarangense* and a product obtained thereby. Such method includes mixing *Syzygium samarangense* and water, adjusting the pH of the resulting mixture to 3. 5 using hydrochloric acid, and conducting an enzymatic hydrolysis reaction using pepsin. Subsequently, the pH of the resulting pepsin hydrolysate is adjusted to 7.5 using NaOH, and trypsin is employed to conduct an enzymatic hydrolysis reaction. The trypsin hydrolysate thus obtained is subjected to centrifugation, followed by collecting the resulting supernatant and subjecting the same to filtration. The filtrate thus obtained is subjected to a concentration process so as to obtain a processed product. It has been proven by experiments that such processed product is effective in treating lung dryness and coughing, hemorrhoid bleeding, indigestion, and enteritis and dysentery.

WO2005/056031A1 discloses a hot water extract from *Syzygium samarangense* and its uses as a lipase inhibitor effective for inhibiting and preventing obesity and hyperlipidemia.

In spite of the aforesaid documents, the researchers in this field still endeavor look for a more satisfactory bioactive component from *Syzygium samarangense*. In a previous study, the applicants found that a water extract of a fruit material of *Syzygium samarangense* is slightly effective in inhibiting the activity of tyrosinase, enhancing the moisture-retaining capacity of skin, and improving would healing (such finding has never been published). In a further study, the applicants surprisingly observed that a hydrolysate, which is prepared by subjecting a water extract (obtained from a fruit material of *Syzygium samarangense*) to a hydrolysis treatment with bromelain, can more effectively inhibit the activity of tyrosinase, enhance the moisture-retaining capacity of skin, and improve wound healing compared to the water extract from which it is prepared. Thus, it is expected that a hydrolysate of water extract of *Syzygium samarangense* can serve as an active ingredient for a cosmetic or pharmaceutical composition.

## SUMMARY

The invention is defined in the appended claims and any other aspects or embodiments set forth herein are for information only.

Therefore, in a first aspect, this invention provides a process for preparing a hydrolysate of water extract of *Syzygium samarangense,* which comprises:

   subjecting a fruit material of *Syzygi um samarangense* to an extraction treatment with water so as to obtain a water extract; and
   subjecting the water extract to a hydrolysis treatment with bromelain.

In a second aspect, this invention provides a hydrolysate of water extract of *Syzygium samarangense,* which is prepared by a process comprising:

   subjecting a fruit material of *Syzygium samarangense* to an extraction treatment with water so as to obtain a water extract, and

subjecting the water extract to a hydrolysis treatment with bromelain.

In a third aspect, this invention provides a composition comprising the aforementioned hydrolysate of water extract of *Syzygium samarangense.*

In a forth aspect, this invention provides a composition as mentioned above which is for whitening skin.

In a fifth aspect, this invention provides a composition as mentioned above which is for enhancing the moisture-retaining capacity of skin.

In a sixth aspect, this invention provides a composition as mentioned above for use in improving wound healing.

In a seventh aspect, this invention provides a composition as mentioned above for use in retarding skin aging.

This invention further provides a composition as mentioned above for use in whitening skin.

## FIGURE LEGEND

Other features and advantages of the disclosure will become apparent in the following detailed description of the embodiments with reference to the accompanying drawings, of which:

FIG. 1 is a HPLC elution profile of the BHWESS (bromelain hydrolysate of water extract of *Syzygium samarangense*) prepared in Example 1.

FIG. 2 shows the ferrous ion chelating ability of the BHWESS prepared in Example 1 and the APHWESS (animal protease hydrolysate of water extract of *Syzygium samarangense*) prepared in Example 3.

FIG. 3 shows the tyrosinase activity inhibitory effect of the BHWESS and WESS (water extract of *Syzygium samarangense*) prepared in Example 1, in which the symbol "###" represents $p<0.001$ (compared with the control sample), and the symbol "***" represents $p<0.001$ (compared with the WESS sample).

FIG. 4 shows the improvement effect of the BHWESS and WESS prepared in Example 1 on wound healing, in which the symbol "##" represents $p<0.01$ (compared with the control group), and the symbol " * *" represents $p< 0.01$ (compared with the WESS group).

FIG. 5 shows the improvement effect of the BHWESS prepared in Example 1 and the APHWESS prepared in Example 3 on wound healing, in which the symbol "###" represents $p<0.001$ (compared with the control group), and the symbol "***" represents $p<0.001$ (compared with the APHWESS group).

FIG. 6 shows the improvement effect of the BHWESS and WESS prepared in Example 1 upon the water content of stratum corneum, in which the symbol "###" represents $p<0.001$ (compared with the control area), and the symbols " * *" and " * * *" respectively represent $p<0.01$ and $p<0.001$ (compared with the WESS area).

FIG. 7 shows the improvement effect of the BHWESS prepared in Example 1 and the APHWESS prepared in Example 3 upon the water content of stratum corneum.

## DETAILLED DESCRIPTION

It is to be understood that, if any prior art publication is referred to herein, such reference does not constitute an admission that the publication forms a part of the common general knowledge in the art, in Taiwan or any other country.

For the purpose of this specification, it will be clearly understood that the word "comprising" means "including but not limited to", and that the word "comprises" has a corresponding meaning.

Unless defined otherwise, all technical and scientific terms used herein have the meaning commonly understood by a person skilled in the art to which the present disclosure belongs.

One skilled in the art will recognize many methods and materials similar or equivalent to those described herein, which could be used in the practice of the present disclosure .

In order to develop a skin care composition that is safe for long term use, the applicants have attempted to obtain extracts of *Syzygium samarangense* fruit using various methods. The applicants have further found that a hydrolysate, which is prepared by subjecting a water extract of *Syzygium samarangense* fruit to a hydrolysis treatment with bromelain, is more effective in performing ferrous ion chelation (i.e. serving as an antioxidant), inhibiting tyrosinase activity, enhancing the moisture-retaining capacity of skin, and improving wound-healing, compared to the water extract from which it is prepared.

Accordingly, the present invention provides a hydrolysate of water extract of *Syzygium samarangense* and a process for preparing the same. The hydrolysate of water extract of *Syzygium samarangense* according to the present invention is prepared by a process comprising:

subjecting a fruit material of *Syzygium samarangense* to an extraction treatment with water so as to obtain a water extract, and
subjecting the water extract to a hydrolysis treatment with bromelain.

According to the present invention, the fruit material of *Syzygium samarangense* comprises a pulp of *Syzygium samarangense.* In some embodiments, the fruit material of *Syzygium samarangense* further comprises a peel of *Syzygium samarangense* and a seed *of Syzygium samarangense.*

According to the present disclosure, the fruit material of *Syzygium samarangense* may be obtained from *Syzygium samarangense* fruits of different cultivars. In some embodiments, the fruit material of *Syzygium samarangense* is obtained from one or more *Syzygium samarangense* fruits belonging to cultivars selected from Black Pearl, dark red cultivars, light red cultivars, pink cultivars (also known as Nanyang cultivars), Black Diamond, and Black King Kong. In an exemplary embodiment, the fruit material of *Syzygium samarangense* is obtained from a *Syzygium samarangense* fruit belonging to the Black Pearl cultivar.

According to the present disclosure, the fruit material of *Syzygium samarangense* may be obtained from *Syzygium samarangense* fruits having various fruit maturity. In certain embodiments, the fruit material of *Syzygium samarangense* is obtained from a young *Syzygium samarangense* fruit. In an exemplary embodiment, the fruit material of *Syzygium samarangense* is obtained from a young *Syzygium samarangense* fruit harvested after one-month of bloom.

According to the present disclosure, the fruit material of *Syzygium samarangense* to be extracted with water may be a fresh fruit material of *Syzygium samarangense,* or may be subjected to a pre-treatment selected from a drying treatment, a grinding treatment, a shredding treatment, a pulverizing treatment, and a combination thereof, prior to the extraction treatment with water.

According to the present disclosure, the extraction treatment with water may be conducted using technology well-known to those skilled in the art. For example, the extraction treatment with water may be conducted according to the method described in the thesis written by Yi-Zhen Chen from the Institute of Cosmetic Science at Chia Nan University of Pharmacy & Science (*supra*).

According to the present invention, the weight ratio of the fruit material of *Syzygium samarangense* to water ranges from 1:0.5 to 1:5. In an embodiment of this disclosure, the weight ratio of the fruit material of *Syzygium samarangense* to water is 1:1.5.

According to the present disclosure, the extraction treatment with water may be conducted at a temperature ranging from 35°C to 65°C. In an embodiment of the present disclosure, the extraction treatment with water may be conducted at 50°C.

It should be noted that the conditions for the extraction treatment with water may vary with several factors (such as the pre-treatment of the fruit material of *Syzygium samarangense,* the weight ratio of the fruit material of *Syzygium samarangense* to water, etc.) so as to achieve a desired extraction result.

According to the present invention, the weight ratio of the water extract to bromelain ranges from 150:1 to 600:1. In an embodiment of the present disclosure, the weight ratio of the water extract to bromelain is 200:1. According to this disclosure, the hydrolysis treatment with bromelain may be conducted at a temperature ranging from 30°C to 60°C for 3 hours to 6 hours. In an embodiment of the present disclosure, the hydrolysis treatment with bromelain is conducted at 45°C for 4 hours.

It should be noted that the conditions for the hydrolysis treatment with bromelain may vary with several factors (such as the weight ratio of the water extract to bromelain, etc.,) so as to achieve the desired hydrolysis result.

According to the present invention, the preparation process for the hydrolysate of water extract of *Syzygium samarangense* may further comprise conducting a solid-liquid separation treatment after the hydrolysis treatment so as to remove a solid portion resulting from the hydrolysis treatment. The solid-liquid separation treatment may be conducted using technology well-known to those skilled in the art. Examples of the solid-liquid separation treatment include, filtration, centrifugation and decantation. In an embodiment of the present disclosure, the solid-liquid separation treatment includes a centrifugation step and a subsequent filtration step.

The applicants have found that the hydrolysate of water extract of *Syzygium samarangense* according to the present invention have the biological effects described below.

First, it has verified via *in vitro* experiments that the hydrolysate of water extract of *Syzygium samarangense* according to the present invention is able to effectively inhibit tyrosinase activity. Therefore, the hydrolysate of water extract of *Syzygium samarangense* according to the present invention is expected to be effective in whitening skin.

As used herein, the terms "whitening skin", "lightening skin color", 'bleaching skin", "brightening skin", "lightening melanin", "inhibiting melanogenesis", and "inhibiting melanin synthesis" can be interchangeably used.

Secondly, the results of the preliminary human tests done by the applicants show that the hydrolysate of water extract of *Syzygium samarangense* according to the present invention can effectively increase the water content of stratum corneum without incurring undesired side effects. Therefore, the hydrolysate of water extract of *Syzygium samarangense* according to this invention is expected to be useful in enhancing the moisture-retaining capacity of skin.

Thirdly, it has been proven via *in vitro* experiments that the hydrolysate of water extract of *Syzygium samarangense* according to the present invention is effective in improving the fibroblast-mediated wound closure . As such, it is contemplated that the hydrolysate of water extract of *Syzygium samarangense* according to this invention can be used in the improvement of wound healing.

Fourthly, it has been verified through *in vitro* experiments that the hydrolysate of water extract of *Syzygium samarangense* according to the present invention has excellent ferrous ion chelating ability. Accordingly, the hydrolysate of water extract of *Syzygium samarangense* according to the present invention can be used as an antioxidant for reducing oxidative stress.

Fifthly, due to the fact that the hydrolysate of water extract of *Syzygium samarangense* according to this invention has antioxidation activity, it can be predicted that the hydrolysate of water extract of *Syzygium samarangense* according to this invention may be effective in preventing and/or retarding skin aging.

As used herein, the term "retarding" refers to treating, reducing, alleviating, ameliorating, relieving, or controlling one or more clinical signs of a disease or disorder, and lowering, stopping, or reversing the progression of severity regarding the condition or symptom being treated.

As used herein, the term "skin aging" is intended to encompass naturally occurring intrinsic skin aging and extrinsic skin aging caused by environmental factors (such as UV radiation). The symptoms of skin aging include, telangiectasia, thinner epidermis, skin atrophy, poor skin texture, dryness, wrinkle formation and pigmentary change (such as lentigines, freckles, hypopigmentation or hyperpigmentation), etc.

Therefore, the present invention provides a composition comprising the aforesaid hydrolysate of water extract of *Syzygium samarangense,* which is suitable for whitening skin, enhancing the moisture-retaining capacity of skin, improving wound healing, reducing oxidative stress, and preventing and/or retarding skin aging.

Further, the present disclosure provides the following uses employing the composition described above.

First, the present invention provides a composition as described above which is for whitening skin. The invention further provides a composition as described above for use in whitening skin. An effective amount of the composition may be administered for a sufficient time until the skin of the subject is significantly whitened. The effective amount of the composition would not cause an adverse side effect to the skin of the subject.

According to the invention, the dosage and frequency of administration of the composition for whitening skin, or for use in whitening skin, may vary with the following factors: the initial condition of the skin area to be subjected to the lightening of melanin, the route of administration, and the desired whitening effect to be achieved. For instance, the dosage of the composition for topical administration according to this invention may be 1-10 $mg/cm^2$ of the skin area, and may be administered one to three times per day. In certain embodiments, the dosage of the composition for oral administration may be 0.05-5 mg/Kg body weight, and may be administered one to three times per day.

Secondly, the present invention provides a composition as described above which is for enhancing the moisture-retaining capacity of skin. An effective amount of the composition may be administered for a sufficient time until the moisture-retaining capacity of the skin of the subject is significantly enhanced. The effective amount of the composition would not cause an adverse side effect to the skin.

According to the invention, the dosage and frequency of administration of the composition for enhancing the moisture-retaining capacity of skin may vary with the following factors: the initial condition of the skin area to be subjected to the enhancement of the moisture-retaining capacity, the route of administration, and the desired moisture-retaining effect to be achieved. For instance, the dosage of the composition for topical administration according to this disclosure may be 1-10 $mg/cm^2$ of the skin area, and may be administered one to three times per day. In certain embodiments, the dosage of the composition for oral administration may be 0.05-5 mg/Kg body weight, and may be administered one to three times per day.

Thirdly, the present invention provides a composition as described above for use in improving wound healing. An effective amount of the composition may be administered for a sufficient time until the wound area is significantly decreased. The effective amount of the composition would not cause an adverse side effect to the wound.

According to the invention, the dosage and frequency of administration of the composition for use in improving wound healing may vary with the following factors: the severity of the wound to be treated, the route of administration, and the desired healing effect to be achieved. For instance, the dosage of the composition for topical administration according to this invention may be 1-10 $mg/cm^2$ of the skin area, and may be administered one to three times per day. In certain embodiments, the dosage of the composition for oral administration may be 0.05-5 mg/Kg body weight, and may be administered one to three times per day.

Fourthly, the present invention provides a composition as described above for use in retarding skin aging, via reducing oxidative stress, which comprises administering to a subject the. An effective amount of the composition may be administered to the subject for a sufficient time until the oxidative stress is significantly reduced. The effective amount of the composition would not cause adverse side effects to the subject.

According to this invention, the dosage and frequency of administration of the composition for use in retarding skin aging, via reducing oxidative stress may vary depending on the following factors: the severity of the oxidative stress to be treated, the route of administration, and the desired antioxidation effect to be achieved. For instance, the dosage of the composition for topical administration according to this disclosure may be 1-10 $mg/cm^2$ of the skin area, and may be administered one to three times per day. The dosage of the composition for oral administration may be 0.05-5 mg/Kg body weight, and may be administered one to three times per day.

Fifthly, the present invention provides a composition as described above for use in preventing and/or retarding skin aging. An effective amount of the composition may be administered to the subject for a sufficient time until at least one symptom of skin aging is significantly improved. The effective amount of the composition would not cause adverse side effects to the subject.

According to the present invention, the dosage and frequency of administration of the composition for preventing and/or retarding skin aging may vary with the following factors: the severity of the symptom of skin aging to be treated, the route of administration, and the desired effect to be achieved. For instance, the dosage of the composition for topical administration according to this invention may be 1-10 mg/cm$^2$ of the skin area, and may be administered one to three times per day. In certain embodiments, the dosage of the composition for oral administration may be 0.05-5 mg/Kg body weight, and may be administered one to three times per day.

Due to the above-mentioned biological activities and safety regarding the hydrolysate of water extract of *Syzygium samarangense,* the composition comprising the same (which is suitable for whitening skin, enhancing the moisture-retaining capacity of skin, for use in improving wound healing, retarding skin aging via reducing oxidative stress, and preventing and/or retarding skin aging) may be a pharmaceutical composition or a cosmetic composition.

The pharmaceutical composition according to the present disclosure can be formulated into a suitable dosage form for oral or topical administration using technology well known to those skilled in the art.

The pharmaceutical composition according to the present disclosure can additionally include a pharmaceutically acceptable carrier widely employed in the art of drug-manufacturing. For instance, the pharmaceutically acceptable carrier may include one or more of the following agents: solvents, buffers, emulsifiers, suspending agents, decomposers, disintegrating agents, dispersing agents, binding agents, excipients, stabilizing agents, chelating agents, preservatives, wetting agents, lubricants, diluents, absorption delaying agents, liposomes, sweetening agents, flavoring agents, coloring agents, etc. The choice and amount of the pharmaceutically acceptable carrier are within the expertise of those skilled in the art.

Examples of the oral dosage form include, aseptic power, tablets, troches, lozenges, pellets, capsules, dispersible powder or granule, solutions, suspensions, emulsions, syrup, elixir, slurry, etc.

Examples of the topical dosage form include, emulsions, gels, ointments, cream, patches, liniments, powder, aerosol, spray, lotions, serum, paste, foam, drops, suspensions, salve, bandages, etc.

In an exemplary embodiment of the present disclosure, the pharmaceutical composition is formulated into an external preparation by admixing the hydrolysate of water extract of *Syzygium samarangense* with a base that is well known and commonly used in the art.

According to this disclosure, suitable bases may include one or more of the following additives: water, alcohol, glycol, hydrocarbons (such as petroleum jelly and white petrolatum), wax (such as paraffin and yellow wax), preserving agents, antioxidants, surfactants, absorption enhancers, stabilizing agents, gelling agents (such as carbopol®974P, microcrystalline cellulose and carboxymethylcellulose), active agents, humectants, odor absorbers, fragrance, pH adjusting agents, chelating agents, emulsifiers, occlusive agents, emollients, thickeners, solubilizing agents, penetration enhancers, anti-irritants, colorants, propellants, etc. The choice and amount of these additives are within the expertise of those skilled in the art.

The cosmetic composition according to the present disclosure may further include a cosmetically acceptable adjuvant that is widely employed in cosmetic-manufacturing technology. For instance, the cosmetically acceptable adjuvant may include one or more of the following agents: solvents, gelling agents, activating agents, preservatives, antioxidants, screening agents, chelating agents, surfactants, coloring agents, thickening agents, fillers, fragrance, and odor absorbents. The choice and amount of these additives are within the expertise of those skilled in the art.

The cosmetic composition of the present disclosure can be prepared using technology well known to a skilled artisan into the form of skincare or makeup products. Such form includes, aqueous solutions, aqueous-alcohol solutions or oily solutions, emulsions, gel, ointments, cream, masks, patches, packs, liniment, powder, aerosol, spray, lotions, serum, paste, foam, suspensions, drops, mousse, sunblock, tonicwater, foundation, eyeshadow, makeup remover products, soaps and other body cleansing products.

The cosmetic composition of the present disclosure can be used with at least one of the following external use agents: whitening agents (such as tretinoin, catechin, kojic acid, arbutin and vitamin C), humectants, anti-inflammatory agents, bactericides, ultraviolet absorbers, algal extracts (such as aloe extracts), skin nutrients, anesthetics, anti-acne agents, antipruritic, analgesic, antiperspirant, antidermatitis agents, antihyperkeratolytic agents, anti-dry skin agents, antiaging agents, antiwrinkle agents, antiseborrheic agents, wound-healing agents, corticosteroid and hormone. The choice and amount of these agents are within the expertise of those skilled in the art.

The invention will be further described by way of the following examples . However, it should be understood that the following examples are solely intended for the purpose of illustration.

**EXAMPLES:**

**Example 1 (illustrative Example). Preparation of bromelain hydrolysate of water extract of *Syzygium samarangense***

3 to 6 g of a fruit material (containing a peel, a pulp, and a seed) of *Syzygium samarangense* harvested from Fangliau Township (Pingtung County, Taiwan) was pulverized. The resulting pulverized product and water were mixed in a weight ratio of 1: 1.5, followed by heating at 50°C for 1 hour. A water extract of *Syzygium samarangense* (referred to as WESS hereinafter) was hence obtained.

Subsequently, the WESS and 1600 IU/g bromelain (NEO-ONE BIOTECH CO. , LTD. ; Cat. No . : 1600 GD) were mixed in a weight ratio of 200:1. The enzymatic hydrolysis reaction was allowed to proceed for 4 hours at 45°C. The reaction mixture thus obtained was subjected to centrifugation, followed by collecting the resulting supernatant. Filters with pore sizes of 5 $\mu$m, 1$\mu$m, and 0.5 $\mu$m (Tech Seed Enterprise Co., Ltd.; Cat. Nos.: PureMax, SCB2000-001, and SP2000-00A) were used sequentially to conduct filtration. The resulting filtrate was subjected to lyophilization, such that a bromelain hydrolysate of water extract of *Syzygium samarangense* (referred to as BHWESS hereinafter) in lyophilized powder form was obtained.

In addition, for the purpose of comparison with the BHWESS, the WESS was also subjected to the centrifugation, filtration, and lyophilization treatments described above so as to convert the WESS into powder form.

**Example 2 (illustrative Example).HPLC analysis for bromelain hydrolysate of water extract of *Syzygium samarangense***

In order to determine the major components of the BHWESS according to the present invention, the BHWESS obtained in Example 1 was dissolved in distilled deionized water so as to prepare a test sample having a BHWESS concentration of 1 mg/mL. The test sample was subjected to a HPLC (high performance liquid chromatography) analysis.

The HPLC instruments employed are as follows: HP Agilent 1100 series HPLC System equipped with a UV-Vis detector and a C18 column (Purospher® STAR RP-18, Cat. No.: 150359, size: 250 mm $\times$ 4.6 mm).

The HPLC operating conditions applied are described as follows. The mobile phase is acetonitrile/deionized water (100:0, v/v), and the flow rate of the mobile phase is 0.7 mL/min. The gradient elution with the mobile phase was conducted for 35 minutes as follows: acetonitrile was decreased from 100% to 90% during 0-10 minutes, was decreased from 90% to 80% during 10-30 minutes, was increased from 80% to 100% at 30 minute, and was maintained at 100% during 30-35 minutes.

*Results:*

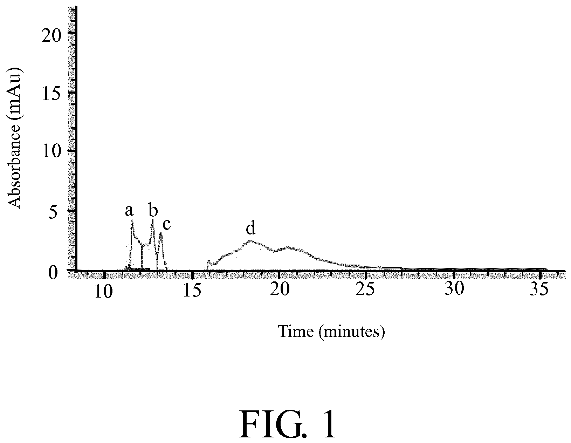

FIG. 1 is a HPLC elutionprofile of the BHWESS prepared in Example 1. As shown in Figure 1, four major peaks (respectively labeled with a, b, c and d) can be observed between a retention time of 0 minute and a retention time of 35 minutes.

**Example 3 (illustrative Example). Evaluation of antioxidation efficacy for bromelain hydrolysate of water extract of *Syzygium samarangense***

In order to examine whether the BHWESS according to the present invention has an antioxidation effect, the ferrous ion chelating ability of the BHWESS prepared in Example 1 was analyzed. In addition, for the sake of comparison with the BHWESS, an animal protease hydrolysate of water extract of *Syzygium samarangense* (referred to as APHWESS hereinafter) was prepared generally according to the method described in CN 102228207 A (with slight modification applied thereto), and was subjected to the same experiment as the BHWESS according to the present invention. The preparation of the APHWESS is described in detail below before the experimental procedures are recited.

20 g of a fruit material (containing a peel, a pulp, and a seed) of *Syzygium samarangense* harvested from Fangliau Township (Pingtung County, Taiwan) was pulverized. The resulting pulverized product and water were mixed in a weight ratio of 1: 1.5, followed by heating at 95°C for 15 minutes. A water extract of *Syzygium samarangense* was hence obtained.

After cooling down to 50°C, the pH of the water extract of *Syzygium samarangense* was adjusted to 3.68 using 4.5% citric acid. Subsequently, the water extract of *Syzygium samarangense* and 2500 IU/mg pepsin (Sigma, Product Number P7012) were mixed in a weight ratio of 50000 : 1. The enzymatic hydrolysis reaction was allowed to proceed for 2 hours at 50°C.

The pH of the resulting pepsin hydrolysate was adjusted to 7.65 using 4 g/mL potassium hydroxide, followed by heating at 95°C for 15 minutes. The pepsin hydrolysate and 2000 IU/mg trypsin (Himedia, Product Number RM618) were mixed in a weight ratio of 27000:1. The enzymatic hydrolysis reaction was allowed to proceed for 3 hours at 50°C.

The pH of the trypsin hydrolysate thus obtained was adjusted to 6.28 using 4.5% citric acid, followed by centrifugation. The resulting supernatant was collected. Filters with pore sizes of 5 $\mu$m, 1 $\mu$m, and 0.5 $\mu$m were used sequentially to conduct filtration. The filtrate thus obtained was subjected to lyophilization, such that the APHWESS in lyophilized powder form was obtained.

*Experimental Procedures:*

A suitable amount of the BHWESS prepared in Example 1 was dissolved in distilled deionized water, such that a BHWESS sample having a BHWESS concentration of 0.35% (w/w) was formed. 1 mL of the BHWESS sample, 3.7 mL of ethanol, and 0.1 mL of a 2 mM $FeCl_2 \cdot 4H_2O$ solution were mixed for 30 seconds. The resulting mixture was uniformly mixed with 0.2 mL of 5 mM ferrozine, followed by incubation in the absence of light at room temperature for 10 minutes. The absorbance at 562 nm ($OD_{562}$) was measured using Colibri Spectrophotometer (Titertek-Berthold). Moreover, 1 mL of a 95% ethanol solution (serving as a control sample) was also subjected to the aforementioned experimental procedures. In addition, a suitable amount of the aforesaid APHWESS prepared generally according to the method described in CN 102228207 A was dissolved in distilled deionized water, such that an APHWESS sample having an APHWESS concentration of 0.35% (w/w) was formed. The APHWESS sample was subjected to the same experimental procedures as the BHWESS sample so as to analyze the ferrous ion chelating ability of the APHWESS.

A lower $OD_{562}$ value indicates that the sample tested has a stronger antioxidation effect.

The percent ferrous ion chelation (%) was calculated using the following Equation (I):

$$A = [1-(B/C)] \times 100 \qquad (I)$$

where A=the percent ferrous ion chelation (%)

B = $OD_{562}$ of a respective one of the BHWESS, APHWESS, and control samples
C= $OD_{562}$ of the control sample

All the experiments were repeated thrice. The experimental data are expressed as mean $\pm$ SD (standard deviation) .

*Results:*

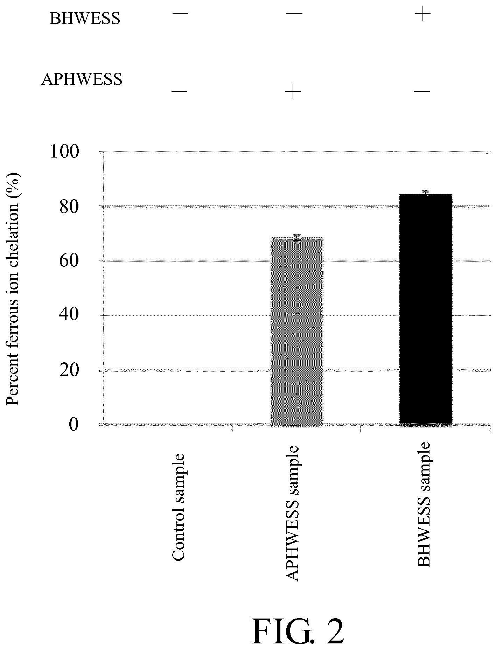

FIG. 2 shows the percent ferrous ion chelation of the BHWESS sample, the control sample, and the APHWESS sample. As shown in FIG. 2, the percent ferrous ion chelation of the BHWESS sample is significantly higher than that of the control sample and the APHWESS sample. Based on the result, the applicants deem that the hydrolysate of water extract of *Syzygium samarangense* according to the present invention is able to exhibit a satisfactory antioxidation effect, even when compared to the animal protease hydrolysate of water extract of *Syzygium samarangense.*

**Example 4 (illustrative Example). Evaluation of melanogenesis inhibitory effect for bromelain hydrolysate of water extract of *Syzygium samarangense***

In this example, the melanogenesis inhibitory effect of the BHWESS according to the present invention was evaluated based on tyrosinase activity. Furthermore, for the purpose of comparison, the WESS prepared in Example 1 was subjected to the same experiment as the BHWESS.

*Experimental Procedures:*

500 mL of a 0.1 M $Na_2HPO_4$ solution and 500 mL of a $KH_2PO_4$ solution were mixed, followed by adjusting the pH of the resulting phosphate buffer solution to 6.8 with NaOH. A suitable amount of tyrosine (Alfa Aesar, Lot: 10174330) was added into a suitable amount of the phosphate buffer solution so as to prepare a 2 mM tyrosine solution.

Subsequently, a suitable amount of a respective one of the BHWESS and WESS prepared in Example 1 was added into a suitable amount of the phosphate buffer solution, such that a BHWESS sample having a BHWESS concentration of 10 mg/mL and a WESS sample having a WESS concentration of 10 mg/mL were obtained. 21.6 $\mu$L of a respective one of the BHWESS and WESS samples was well mixed with 250 $\mu$L of tyrosinase (Sigma, Lot: SLBJ5647V) (115.42 U/mL, in a suitable amount of the phosphate buffer solution) and 540 $\mu$L of the tyrosine solution. The resultant mixture was incubated at 37°C in the absence of light for 30 minutes . Afterward, the absorbance at 475 nm ($OD_{475}$)

was measured using the spectrophotometer. Furthermore, a 290 μL of the phosphate buffer solution (serving as a control sample) was also subjected to the aforementioned experimental procedures. A lower $OD_{475}$ value indicates that the sample tested has a stronger tyrosinase inhibitory activity.

The percent tyrosinase activity inhibition (%) was calculated using the following Equation (II):

$$D = [1-(E/F)] \times 100 \qquad (II)$$

where D = the percent tyrosinase activity inhibition (%)

E=$OD_{475}$ of a respective one of the BHWESS, WESS and control samples
F=$OD_{475}$ of the control sample

All the experiments were repeated twice. The experimental data are expressed as mean $\pm$ SD, and were analyzed by virtue of Student's test so as to assess the difference between the BHWESS sample, the WESS sample, and the control sample. Statistical significance is indicated by $p<0.05$.

*Results:*

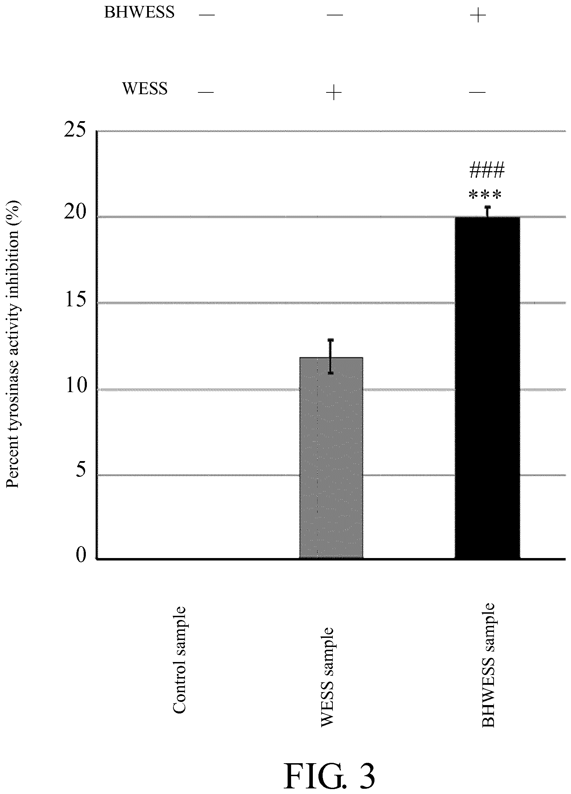

FIG. 3 shows the percent tyrosinase activity inhibition of the BHWESS sample, the WESS sample, and the control sample. As shown in FIG. 3, the percentage percent tyrosinase activity inhibition of the BHWESS sample is significantly higher than that of each of the WESS sample and control sample. The experimental result indicates that the hydrolysate of water extract of *Syzygium samarangense* according to the present invention has a better tyrosinase inhibitory ability compared to the water extract of *Syzygium samarangense.* Accordingly, the applicants deem that the hydrolysate of water extract of *Syzygium samarangense* according to the present invention can inhibit melanogenesis by suppressing tyrosinase activity, thereby having a skin whitening effect.

**Example 5 (illustrative Example). Evaluation of improvement effect on wound healing for bromelain hydrolysate of water extract of *Syzygium samarangense***

In this example, the improvement effect of the BHWESS according to the present invention on wound healing was evaluated by virtue of wound-healing cell migration assay. In addition, for the purpose of comparison, the WESS prepared in Example 1 and the APHWESS prepared in Example 3 were both subjected to the same evaluation.

*Experimental Materials:*

**1. Human skin fibroblast cell line Detroit 551**

Human skin fibroblast cell line Detroit 551 used in this example was purchased from Biosource Collection and Research Center (BCRC) of Food Industry Research and Development Institute (FIRDI) (331 Shih-Pin Road, Hsinchu 300, Taiwan).

Detroit 551 cells were incubated in a Petri dish containing a minimum essential medium (MEM; Sigma, Product No. : M2279) supplemented with 10% fetal bovine serum (FBS) and 10% penicillin-streptomycin (Gibco™, Cat. No.: 15140122), followed by cultivation in an incubator (37°C, 5% $CO_2$) . Medium change was performed every two days. Cell passage was performed when the cultured cells reached 80%-90% of confluence.

*Experimental Procedures:*

**A. Comparison of improvement effect on wound healing between BHWESS and WESS**

The Detroit 551 cells obtained in section 1 of Experimental Materials were divided into 3 groups including a BHWESS group, a WESS group, and a control group. Each group of the Detroit 551 cells was incubated in a 24-well plate containing 500 μL of MEM (supplemented with 10% FBS) at $2 \times 10^5$ cells/well, followed by cultivation in an incubator (37°C, 5% $CO_2$) for 24 hours.

After medium change with a fresh medium, a plastic pipette tip of 200 μL was used to scratch the Detroit 551 cells in each well of the plate along a diametral line. Therefore, a wound zone having a width of 500 μm and no cells attached thereto was formed along a diametral line of each well. Washing was conducted with phosphate buffered saline (PBS) twice. After medium change was performed with a fresh MEM (supplemented with 5% FBS), the cell cultures of the BHWESS and WESS groups were respectively treated with suitable amounts of the BHWESS and WESS prepared in Example 1, so that the cell cultures of the BHWESS and WESS groups respectively had final concentrations of 0.03% (w/w) BHWESS and 0.03% (w/w) WESS. The cell culture of the control group received no treatment.

Subsequently, cultivation was conducted in an incubator (37°C, 5% $CO_2$) for 12 hours. At 0 hour (i.e. before the treatment) and 12 hour after the treatment, the wound zones of each group were observed using an inverted microscope (NIKON TE300, Japan) under 40X magnification, were photographed using a digital camera (Nikon F90, Japan), and were subjected to area calculation according to the photographs obtained using ImageJ software.

The wound healing percentage (%) was calculated using the following Equation (III):

$$G = [1-(H/I)] \times 100 \qquad (III)$$

where G=the wound healing percentage (%)

H=the area of the wound zone at 12 hour after the treatment

I=the area of the wound zone at 0 hour

All experiments were repeated twice. The experimental data are expressed as mean $\pm$ SD, and were analyzed by virtue of Student's test so as to assess the difference between the BHWESS group, the WESS group, and the control group. Statistical significance is indicated by $p<0.05$.

**B. Comparison of improvement effect on wound healing between BHWESS and APHWESS**

First of all, SPLScar™ Block (Bio-genesis Technologies, Inc., Catalog Number 201902) having a width of 500 $\mu$m was placed along a diametral line of each well of a 24-well plate. Subsequently, the Detroit 551 cells obtained in section 1 of Experimental Materials were divided into 3 groups including a BHWESS group, an APHWESS group, and a control group. Each group of the Detroit 551 cells was incubated in the 24-wellplate containing 500 $\mu$L of MEM (supplemented with 10% FBS) at $5 \times 10^4$ cells/well, followed by cultivation in an incubator (37°C, 5% $CO_2$) for 24 hours.

Afterward, SPLScar™ Block was removed so as to form a wound zone having a width of 500 $\mu$m and no cells attached thereto. Washing was conducted with PBS twice. After medium change was performed with a fresh MEM (supplemented with 5% FBS), the cell cultures of the BHWESS and APHWESS groups were respectively treated with suitable amounts of the BHWESS prepared in Example 1 and the APHWESS prepared in Example 3, so that the cell cultures of the BHWESS and APHWESS groups respectively had final concentrations of 0.025% (w/w) BHWESS and 0.025% (w/w) APHWESS. The cell culture of the control group received no treatment.

Subsequently, cultivation was conducted in an incubator (37°C, 5% $CO_2$) for 12 hours. At 0 hour (i.e. before the treatment) and 12 hour after the treatment, the wound zones of each group were observed using an inverted microscope (Olympus IX73) under 40X magnification, were photographed using a digital camera (Olympus DP80), and were subjected to area calculation according to the photographs obtained using ImageJ software. The wound healing percentage (%) was calculated using Equation (III).

All experiments were repeated thrice. The experimental data are expressed as mean $\pm$ SD, and were analyzed by virtue of Student's test so as to assess the difference between the BHWESS group, the APHWESS group, and the control group. Statistical significance is indicated by $p<0.05$.

***Results:***

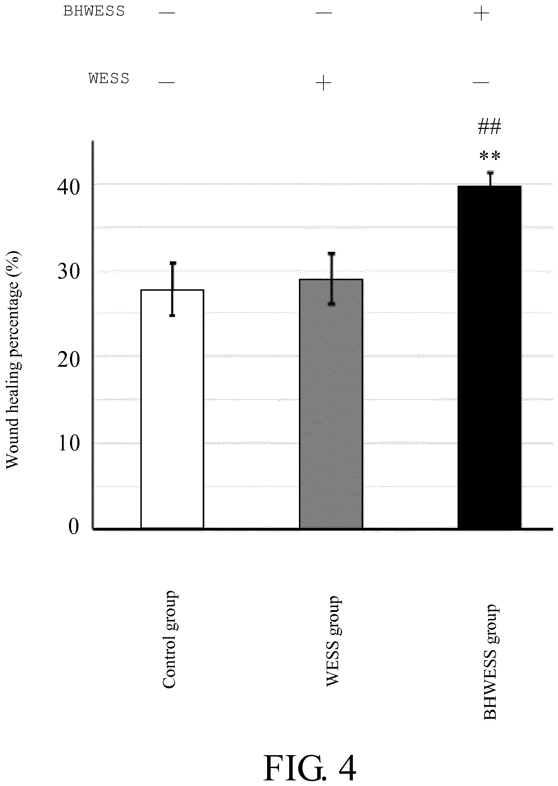

FIG. 4 shows the wound healing percentage of the Detroit 551 cells treated with BHWESS and WESS, and those receiving no treatment. As shown in FIG. 4, there is no significant difference in the wound healing percentage between the WESS group and the control group. However, the wound healing percentage of the BHWESS group is significantly higher than that of each of the control group and the WESS group.

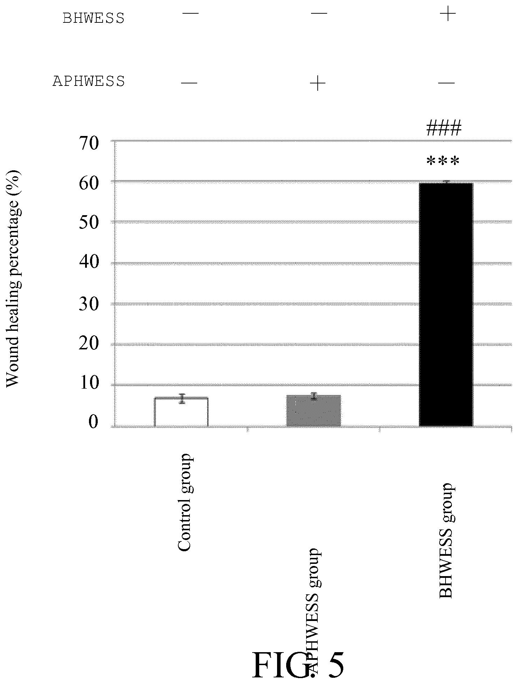

FIG. 5 shows the wound healing percentage of the Detroit 551 cells treated with BHWESS and APHWESS, and those receiving no treatment. As shown in FIG. 5, there is no significant difference in the wound healing percentage between the APHWESS group and the control group. However, the wound healing percentage of the BHWESS group is significantly higher than that of each of the control group and the APHWESS group.

The aforesaid experimental results reveal that the hydrolysate of water extract of *Syzygium samarangense* according to the present invention is effective in improving fibroblast-mediated wound closure, thereby being able to facilitate wound healing.

**Example 6 (illustrative Example). Evaluation of enhancement effect on moisture-retaining capacity of skin for bromelain hydrolysate of water extract of _Syzygium samarangense_**

In order to investigate the enhancement effect of the BHWESS according to this invention on the moisture-retaining capacity of human skin, the following experiments were performed.

**A. Preparation of essences**

The BHWESS and the WESS prepared in Example 1, as well as the APHWESS prepared in Example 3, were used in this example. Four essences (including a BHWESS essence, a WESS essence, an APHWESS essence, and a control essence) were respectively formulated using the recipes shown in Table 1.

Table 1

| Ingredients | BHWESS essence | WESS essence | APHWESS essence | Control essence |
|---|---|---|---|---|
| Butylene glycol# | 1 | 1 | 1 | 1 |
| Hydroxyethyl cellulose# | 0.3 | 0.3 | 0.3 | 0.3 |
| Dermosoft OMP* | 3 | 3 | 3 | 3 |
| BHWESS | 4 | - | - | - |
| WESS | - | 4 | - | - |
| APHWESS | - | - | 4 | - |
| Deionized water | 91.7 | 91.7 | 91.7 | 95.7 |
| The amount of each ingredient is expressed as % (w/w).<br>#: purchased from First Chemical Co., Ltd.<br>*: purchased from HonorChem Co., Ltd. | | | | |

The process for formulating the BHWESS essence, the WESS essence, and the APHWESS essence is described in more detail as follows. Butylene glycol and hydroxyethyl cellulose were dissolved in deionized water at 75°C under agitation, followed by cooling to 40°C-45°C. A respective one of the BHWESS, the WESS, and the APHWESS was added, followed by mixing with Dermosoft OMP under agitation. Then, NaOH was used to adjust the pH of the resulting mixture to about 6 to 7. Thus, the BHWESS essence, the WESS essence, and the APHWESS essence were obtained.

The process for formulating the control essence is similar to that for formulating the BHWESS essence, except that additional deionized water was used instead of the BHWESS.

**B. Comparison of enhancement effect on moisture-retaining capacity of skin between BHWESS and WESS**

Subjects enrolled from Yeun Diing Enterprise CO., LTD. (Pingtung county, Taiwan) and M.H. Biotechnology Co., Ltd. (Yunlin County, Taiwan) were subjected to screening according to the exclusion criteria outlined in Table 2. A total of 10 eligible test subjects, including 4 males and 6 females at an age ranging from 25 to 40 years, participated in the following test.

Table 2

| No. | Exclusion criteria |
|---|---|
| 1 | Possession of sensitive skin |
| 2 | Possession of a skin disease, such as eczema, atopic dermatitis and allergic dermatitis |
| 3 | Pregnancy |
| 4 | Use of other skin care product(s) containing a moisture-retaining ingredient during the test |

The right arm of each of the test subjects was randomly divided into three test areas having a width of 4 fingerbreadths (i.e. a BHWESS area, a WESS area and a control area), onto which the BHWESS essence, the WESS essence, and the control essence prepared in section A of this example were respectively applied at a dose of 0.5 mL twice daily. The test period for each test area lasted for a total of 28 days.

On Day 0 (i.e. prior to the application of the test essences) and at designated time points after the beginning of the application of the test essences (Days 7, 14, 21 and 28), the test areas on each test subject were separately subjected to determination of the water content of stratum corneum using a capacitance meter (Corneometer® CM 285, Courage & Khazaka).

The percent improvement of the water content of stratum corneum (%) was calculated using the following Equation (IV):

$$J = [(L-K)/K] \times 100 \qquad (IV)$$

where J=the percent improvement of the water content of stratum corneum (%) at the respective designated time point (Day 7, Day 14, Day 21 or Day 28)
K=the water content of stratum corneum on Day 0
L=the water content of stratum corneum at the respective designated time point

The test areas on each test subject were repeatedly subjected to the water content determination 3 times. The experimental data are expressed as mean ± SD, and were analyzed by virtue of Student's test so as to assess the difference between the test areas. Statistical significance is indicated by $p < 0.05$.

In addition, safety evaluation was conducted by a researcher on Day 0 and at the designated time points thereafter (Days 7, 14, 21 and 28) . On Day 0, small amounts of the essences were applied onto an inner side of an upper arm portion for safety evaluation only. The test subjects were surveyed by the researcher in respect to the occurrence of any adverse skin response (including itchiness, irritation and erythema).

### C. Comparison of enhancement effect on moisture-retaining capacity of skin between BHWESS and APHWESS

Subjects enrolled from M.H. Biotechnology Co., Ltd. were subjected to screening according to the exclusion criteria outlined in Table 2. A total of 9 eligible test subjects, including 6 males and 3 females at an age ranging from 25 to 40 years, participated in the following test.

The application of test essences, determination of the water content of stratum corneum, and safety evaluation were conducted generally according to the procedures described in section B of this example, except the following: the APHWESS essence was tested instead of the WESS essence; the area onto which the APHWESS essence was applied is referred to as an APHWESS area (i.e. the APHWESS area replaced the WESS area) ; and the determination of the water content of stratum corneum was conducted at only one designated time point (Day 14) after the beginning of the application of the test essences.

### *Results:*

### 1. Determination of water content of stratum corneum

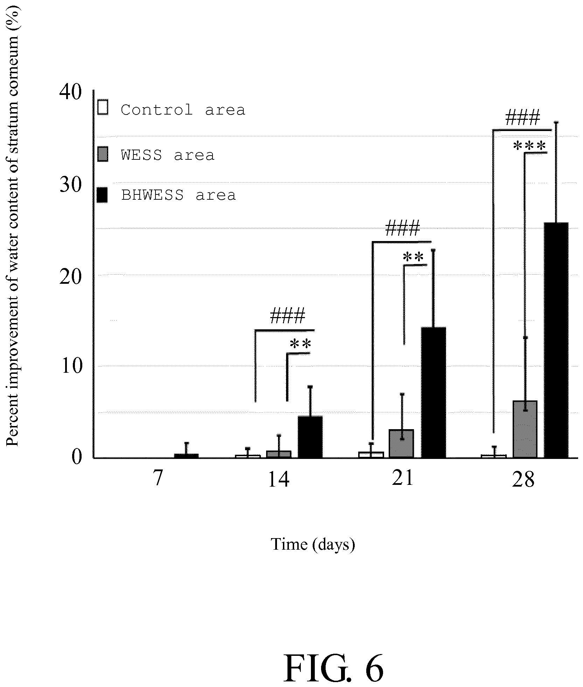

FIG. 6 shows the percent improvement of the water content of stratum corneum in each of the BHWESS, WESS and control areas at different time points. As shown in FIG. 6, on Days 14, 21 and 28 after the beginning of the application of the test essences, the percent improvement of the water content of stratum corneum in the BHWESS area is significant higher than that in each of the WESS area and the control area. In addition, the water content of stratum corneum in the BHWESS area drmatically increased with time, while the improvement of water content in the WESS test area was quite slow.

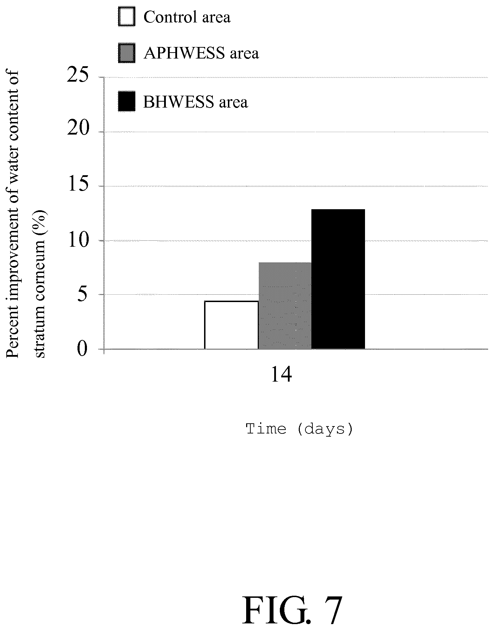

FIG. 7 shows the percent improvement of the water content of stratum corneum in each of the BHWESS, APHWESS and control areas on Day 14. As shown in FIG. 7, the percent improvement of the water content of stratum corneum in the BHWESS area is higher than that in each of the APHWESS area and the control area.

The aforesaid data suggest that the hydrolysate of water extract of *Syzygium samarangense* according to the present invention can be used to effectively enhance the moisture-retaining capacity of skin.

### 2. Safety evaluation

During the test period, no adverse skin response was observed on all the test subjects (data not shown).

In view of the foregoing, the hydrolysate of water extract of *Syzygium samarangense* according to the present invention can exhibit an excellent antioxidationactivity, inhibit melanogenesis, enhance the moisture-retaining capacity of skin, and promote wound healing. Therefore, the applicants deem that the hydrolysate of water extract of *Syzygium samarangense* according to the present invention can be developed into a skin care product for long-term use which does not induce adverse side effects.

**Claims**

1. A process for preparing a hydrolysate of water extract of *Syzygium samarangense,* **characterized by**:

   subjecting a fruit material of *Syzygium samarangense* to an extraction treatment with water so as to obtain a water extract; and
   subjecting the water extract to a hydrolysis treatment with bromelain.

2. The process of Claim 1, **characterized in that** the weight ratio of the fruit material of *Syzygium samarangense* to water ranges from 1:0.5 to 1:5.

3. The process of Claim 1 or Claim 2, **characterized in that** the weight ratio of the water extract to bromelain ranges from 150:1 to 600:1.

4. The process of any one of Claims 1 to 3, further **characterized by** conducting a solid-liquid separation treatment after the hydrolysis treatment so as to remove a solid portion resulting from the hydrolysis treatment.

5. The process of any one of Claims 1 to 4, **characterized in that** the fruit material of *Syzygium samarangense* comprises a pulp of *Syzygium samarangense.*

6. The process of Claim 5, **characterized in that** the fruit material of *Syzygium samarangense further* comprises a peel of *Syzygium samarangense* and a seed *of Syzygium samarangense.*

7. A hydrolysate of water extract of *Syzygium samarangense,* which is prepared by a process **characterized by**:

   subjecting a fruit material of *Syzygium samarangense* to an extraction treatment with water so as to obtain a water extract; and
   subjecting the water extract to a hydrolysis treatment with bromelain.

8. The hydrolysate as claimed in Claim 7, **characterized in that** the weight ratio of the fruit material of the *Syzygium samarangense* to water ranges from 1:0.5 to 1:5.

9. The hydrolysate as claimed in Claim 7 or Claim 8, **characterized in that** the weight ratio of the water extract to bromelain ranges from 150:1 to 600:1.

10. The hydrolysate as claimed in any one of Claims 7 to 9, **characterized in that** the process further comprises conducting a solid-liquid separation treatment after the hydrolysis treatment so as to remove a solid portion resulting from the hydrolysis treatment.

11. The hydrolysate as claimed in any one of Claims 7 to 10, **characterized in that** the fruit material of *Syzygium samarangense* comprises a pulp of *Syzygium samarangense.*

12. The hydrolysate as claimed in Claim 11, **characterized in that** the fruit material of *Syzygium samarangense further* comprises a peel of *Syzygium samarangense* and a seed *of Syzygium samarangense.*

13. A composition **characterized by** a hydrolysate according to any one of Claims 7 to 12.

14. The composition as claimed in Claim 13, which is for whitening skin and/or enhancing the moisture-retaining capacity of skin.

15. The composition as claimed in claim 13 for use in improving wound healing, in whitening skin, and/or in retarding skin aging.

**Patentansprüche**

1. Verfahren zur Herstellung eines Hydrolysats aus Wasserextrakt von *Syzygium samarangense,* **gekennzeichnet durch**:

   Unterziehen eines Fruchtmaterials von *Syzygium samarangense* einer Extraktionsbehandlung mit Wasser, um einen Wasserextrakt zu erhalten; und
   Unterziehen des Wasserextrakts einer Hydrolysebehandlung mit Bromelain.

2. Verfahren nach Anspruch 1, **dadurch gekennzeichnet, dass** das Gewichtsverhältnis des Fruchtmaterials von *Syzygium samarangense* zu Wasser zwischen 1:0,5 und 1:5 liegt.

3. Verfahren nach Anspruch 1 oder Anspruch 2, **dadurch gekennzeichnet, dass** das Gewichtsverhältnis des Wasserextrakts zu Bromelain zwischen 150:1 und 600:1 liegt.

4. Verfahren nach einem der Ansprüche 1 bis 3, ferner **gekennzeichnet durch** das Durchführen einer Fest-Flüssig-Trennbehandlung nach der Hydrolysebehandlung, um einen aus der Hydrolysebehandlung resultierenden festen Anteil zu entfernen.

5. Verfahren nach einem der Ansprüche 1 bis 4, **dadurch gekennzeichnet, dass** das Fruchtmaterial von *Syzygium samarangense* ein Fruchtfleisch von *Syzygium samarangense* umfasst.

6. Verfahren nach Anspruch 5, **dadurch gekennzeichnet, dass** das Fruchtmaterial von *Syzygium samarangense* ferner eine Schale von *Syzygium samarangense* und einen Samen von *Syzygium samarangense* umfasst.

7. Hydrolysat aus Wasserextrakt von *Syzygium samarangense,* welches mit einem Verfahren hergestellt wird, das **gekennzeichnet ist durch**:

   Unterziehen eines Fruchtmaterials von *Syzygium samarangense* einer Extraktionsbehandlung mit Wasser, um einen Wasserextrakt zu erhalten; und
   Unterziehen des Wasserextrakts einer Hydrolysebehandlung mit Bromelain.

8. Hydrolysat nach Anspruch 7, **dadurch gekennzeichnet, dass** das Gewichtsverhältnis des Fruchtmaterials von *Syzygium samarangense* zu Wasser zwischen 1:0,5 und 1:5 liegt.

9. Hydrolysat nach Anspruch 7 oder Anspruch 8, **dadurch gekennzeichnet, dass** das Gewichtsverhältnis des Wasserextrakts zu Bromelain zwischen 150:1 und 600:1 liegt.

10. Hydrolysat nach einem der Ansprüche 7 bis 9, **dadurch gekennzeichnet, dass** das Verfahren ferner das Durchführen einer Fest-Flüssig-Trennbehandlung nach der Hydrolysebehandlung umfasst, um einen aus der Hydrolysebehandlung resultierenden festen Teil zu entfernen.

11. Hydrolysat nach einem der Ansprüche 7 bis 10, **dadurch gekennzeichnet, dass** das Fruchtmaterial von *Syzygium samarangense* ein Fruchtfleisch von *Syzygium samarangense* umfasst.

12. Hydrolysat nach Anspruch 11, **dadurch gekennzeichnet, dass** das Fruchtmaterial von *Syzygium samarangense* ferner eine Schale von *Syzygium samarangense* und einen Samen von *Syzygium samarangense* umfasst.

13. Zusammensetzung **gekennzeichnet durch** ein Hydrolysat nach einem der Ansprüche 7 bis 12.

14. Zusammensetzung nach Anspruch 13, die zur Aufhellung der Haut und/oder zur Verbesserung des Feuchtigkeitsbindevermögens der Haut dient.

15. Zusammensetzung nach Anspruch 13 zur Verwendung bei der Verbesserung der Wundheilung, bei der Aufhellung der Haut und/oder bei der Verzögerung der Hautalterung.

**Revendications**

1. Procédé pour préparer un hydrolysat d'extrait aqueux de *Syzygium samarangense,* **caractérisé par** :

   la soumission d'un matériau fruitier de *Syzygium samarangense* à un traitement d'extraction avec de l'eau de façon que soit obtenu un extrait aqueux ; et
   la soumission de l'extrait aqueux à un traitement d'hydrolyse avec de la broméline.

2. Procédé selon la revendication 1, **caractérisé en ce que** le rapport en poids du matériau fruitier de *Syzygium samarangense* à l'eau est situé dans la plage allant de 1/0,5 à 1/5.

3. Procédé selon la revendication 1 ou la revendication 2, **caractérisé en ce que** le rapport en poids de l'extrait aqueux à la broméline est situé dans la plage allant de 150/1 à 600/1.

4. Procédé selon l'une quelconque des revendications 1 à 3, **caractérisé en outre par** la mise en œuvre d'un traitement de séparation solide-liquide après le traitement d'hydrolyse de façon à éliminer une partie solide résultant du traitement d'hydrolyse.

5. Procédé selon l'une quelconque des revendications 1 à 4, **caractérisé en ce que** le matériau fruitier de *Syzygium samarangense* comprend de la pulpe de *Syzygium samarangense.*

6. Procédé selon la revendication 5, **caractérisé en ce que** le matériau fruitier de *Syzygium samarangense* comprend en outre des peaux de *Syzygium samarangense* et des graines de *Syzygium samarangense.*

7. Hydrolysat d'extrait aqueux de *Syzygium samarangense,* qui est préparé par un procédé **caractérisé par** :

   la soumission d'un matériau fruitier de *Syzygium samarangense* à un traitement d'extraction avec de l'eau de façon que soit obtenu un extrait aqueux ; et
   la soumission de l'extrait aqueux à un traitement d'hydrolyse avec de la broméline.

8. Hydrolysat selon la revendication 7, **caractérisé en ce que** le rapport en poids du matériau fruitier de *Syzygium samarangense* à l'eau est situé dans la plage allant de 1/0,5 à 1/5.

9. Hydrolysat selon la revendication 7 ou la revendication 8, **caractérisé en ce que** le rapport en poids de l'extrait aqueux à la broméline est situé dans la plage allant de 150/1 à 600/1.

10. Hydrolysat selon l'une quelconque des revendications 7 à 9, **caractérisé en ce que** le procédé comprend en outre la mise en œuvre d'un traitement de séparation solide-liquide après le traitement d'hydrolyse de façon à éliminer une partie solide résultant du traitement d'hydrolyse.

11. Hydrolysat selon l'une quelconque des revendications 7 à 10, **caractérisé en ce que** le matériau fruitier de *Syzygium samarangense* comprend de la pulpe de *Syzygium samarangense.*

12. Hydrolysat selon la revendication 11, **caractérisé en ce que** le matériau fruitier de *Syzygium samarangense* comprend en outre des peaux de *Syzygium samarangense* et des graines de *Syzygium samarangense.*

13. Composition **caractérisée par** un hydrolysat selon l'une quelconque des revendications 7 à 12.

14. Composition selon la revendication 13, qui est pour blanchir la peau et/ou amplifier la capacité de rétention d'humidité de la peau.

15. Composition selon la revendication 13, destinée à être utilisée dans l'amélioration de la cicatrisation, dans le blanchiment de la peau, et/ou dans le retardement du vieillissement de la peau.

FIG. 1

FIG. 2

FIG. 3

FIG. 4

FIG. 5

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- TW 1465258
- CN 102228207 A

- WO 2005056031 A1

**Non-patent literature cited in the description**

- **KIM Y.J. ; YOKOZAWA T.** *Biol. Pharm. Bull.,* 2009, vol. 32, 1155-1159
- **KURAHASHI T ; FUJII J.** *J. Dev. Biol.,* 2009, vol. 3, 57-70
- **PEI-WEN LO.** Evaluation of the Antioxidative and Free Radical-scavenging Activity of Several Taiwan Unique Fruits. Department of Nutrition and Food Science at Fu-Jen Catholic University

- **YI-ZHEN CHEN.** Application of Extracts from Wax Apple Flowers on Antioxidantion. Institute of Cosmetic Science at Chia NanUniversity of Pharmacy & Science